# EUROPEAN PATENT APPLICATION

(11) **EP 1 621 551 A1**
(43) Date of publication of application: **01.02.2006**
(21) Application number: 04291913.4
(22) Date of filing: 27.07.2004
(51) Int. Cl.: C07K 14/705, A61K 39/395, A61K 38/17, G01N 33/68, A61P 35/00, A61P 37/00

(54) **Use of LLT1 and/or CD161 for modulating the activity of cells of the immune system**

(71) Applicant: CENTRE NATIONAL DE LA RECHERCHE SCIENTIFIQUE, 75794 Paris Cedex 16 (FR)
(72) Inventor: Braud, Véronique Marie, F-06130Grasse (FR); Josso-Aldemir, Hatice 413 La Pinède, F-06160 Antibes-Juan Les Pins (FR); Prod'homme, Virginie, F-44500 La Baule-Escoublac (FR)
(74) Representative: Grosset-Fournier, Chantal Catherine

(57) **Abstract**

The present invention relates to a method for modulating *in vivo* or *in vitro* the activity of cells expressing CD161 and/or LLT1, characterized in that said cells are contacted respectively with LLT1 or fragments thereof, and/or with CD161 or fragments thereof.

## Description

The present invention relates to a method for modulating the activity of cells of the immune system.

Natural killer cells and T cells are key effectors of immune responses. A panel of receptors and costimulatory molecules modulates their activity. T-cells become activated by engagement of their clonotypic T-cell antigen receptor (TCR)-CD3 complexes by specific MHC class I or II-peptide complexes and by engagement of costimulatory receptors by their ligands expressed on professional antigen-presenting cells. The antigen activation threshold can be modulated by NK receptors expressed on their cell surface.

As opposed to T cells, NK cell recognition of targets is not antigen specific and NK cells are capable of killing target cells without prior sensitization. NK cell activity is regulated by a balance between activating and inhibitory signals triggered by engagement of NK cell receptors with their ligands. NK cells generally bind and kill target cells which are deficient in the expression of MHC class I proteins ("missing self' hypothesis) (Llunggren et al, Immunol. Today. 11:237-244, 1990). This is because NK cells express NK cell inhibitory receptors that recognizes MHC class I molecules. They include Killer cell Ig-like Receptors (KIR) interacting with groups of classical MHC class I molecules, CD94/NKG2A recognizing non classical HLA-E molecule and Ig-like transcript 2 (ILT-2) interacting with most if not all MHC class I proteins (Moretta et al, Annu. Rev. Immunol. 19:197-223, 2001). Activation and lysis of target cells is triggered mainly by Natural Cytotoxicity Receptors (NCR) and NKG2D. But a number of other activating receptors or costimulatory molecules also modulate NK cell activation. Only when all receptors and costimulatory molecules and their respective ligands are identified, will it be possible to fully understand the regulation of NK cell activity and their function.

Human Lectin-like transcript 1 (LLT1) has an extracellular domain with similarity to the C-type lectin like domains shared with other NK cell receptors including CD161 (also named NKR-P1A). LLT1 was cloned in 1999 (Genebank accession number AF133299) and is localized to the NK gene complex on human chromosome 12 in between NKR-P1A and CD69 (Boles et al, Immunogenetics. 50:1-7, 1999). Recently, a monoclonal antibody L9.7 (mAb) has been described to specifically recognize LLT1 and to induce IFN-γ cytokine production but not cytotoxicity in YT cells, a human NK cell line but also in resting NK cells freshly isolated from peripheral blood and IL-2 activated NK cells (Mathew PA et al, Mol. Immunol. 40:1157, 2004). L9.7 mAb recognizes most cells from peripheral blood mononuclear cells including NK cells (4-10%), T cells (5-14%), B cells (64-73%) and monocytes (100%). LLT1 is expressed on the cell surface as a homodimer.

CD161 (also named NKR-P1A) is also a C-type lectin expressed by natural killer cells and subsets of T cells, including TCR αβ and γδ T cells, invariant CDld-restricted Vα24⁺ NKT and CD2⁻CD3/TCR⁻thymocytes (Lanier et al, J. Immunol. 153:2417-2428,1994; Poggi et al, Eur. J. Immunol. 26:1266-1272,1996; Exley et al, J. Exp. Med. 188:867-876,1998). CD161 is a disulfide-bond homodimer. Anti-CD161 mAbs have been reported to inhibit the NK cell-mediated lysis of some Fcγ receptor⁺ tumour target cells (Lanier et al, J. Immunol. 153:2417-2428,1994; Poggi et al, Eur. J. Immunol. 28:1611-1616, 1998). CD161 was also shown to act as a costimulatory molecule on CD1d-restricted Vα24⁺ NKT cells, mediating both proliferation and cytokine secretion (Exley et al, J. Exp. Med., 188:867-876,1998). So far, no ligand has been identified for CD161. It was reported that CD161 binds carbohydrates but the authors later retracted the article. Expression of CD161 on the cell surface is upregulated by IL-12 cytokine (Poggi et al, Eur. J. Immunol. 28:1611-1616, 1998). CD161 has also been reported to be involved in transendothelial migration (Poggi et al, Eur. J. Immuol. 27:2345-2350, 1997). It may upregulate LFA1 Mg²⁺ binding site and β1 and β2 (CD29) integrin at the cell surface.

The present invention arises from the unexpected finding made by the Inventors, that CD161 expressing cells could be specifically linked to LLT1.

Thus, an object of the present invention is to provide a new interaction between two surface receptors of cells of the immune system, namely CD161 and LLT1.

Another object of the invention is to use this interaction to modulate the activity of cells of the immune system.

A further object of the invention is to provide pharmaceutical compositions comprising compounds liable to mimic and/or to block the above-defined interaction.

The present invention relates to a method for modulating *in vivo* or *in vitro* the activity of cells expressing CD161 and/or LLT1, characterized in that said cells are contacted respectively with LLT1 or fragments thereof, and/or with CD161 or fragments thereof.

As intended herein "modulating the activity of cells" relates to the activation or to the inhibition of the activity of said cells.

The activity of given cells can be measured according to methods well known to the man skilled in the art. Such methods notably involve measuring the migration, the proliferation, the cytokine production, or the cytotoxicity of said cells.

According to a preferred embodiment of the above-defined method, the CD 161 expressing cells are NK cells, CD1d restricted Vα24⁺ NKT cells, or T cells, such as α*β* T cells or *γδ* T cells.

According to another preferred embodiment of the above-defined method, when the CD161 expressing cells are NK cells, the contacting of said cells with LLT1 or fragments thereof leads to the inhibition of said cells.

Under certain conditions, the contacting of CD161 expressing NK cells with LLT1 or fragments thereof also leads to the activation of said cells.

In another preferred embodiment of the above-defined method, when the CD161 expressing cells are T cells or CD1d restricted Vα24⁺ NKT cells, the contacting of said cells with LLT1 or fragments thereof leads to the activation of said cells.

Under certain conditions, the contacting of CD161 expressing T cells or Vα24⁺ NKT cells with LLT1 or fragments thereof also leads to the inhibition of said cells.

In yet another preferred embodiment of the above-defined method, the CD161 expressing cells are contacted with:
- a human LLT1, in particular as represented by SEQ ID NO: 2, or
- a fragment of said human LLT1 corresponding to the extracellular domains of LLT1, in particular as represented by SEQ ID NO: 4.

Advantageously, extracellular domains of LLT1 are soluble by themselves.

According to a particular embodiment of the above-defined method, LLT1, or fragments thereof, are presented at the surface of biological membranes, and in particular at the surface of cells, are used as soluble molecules, or are immobilized on a support.

By "biological membrane" is meant any membrane constituted of amphiphatic lipids, such as glycerophospholipids.

By "support", is meant any solid material onto which proteins can be attached, either directly or by means of a suitable linker molecule, according to methods well-known to the man skilled in the art.

According to another particular embodiment of the above-defined method, the LLT1 fragment is part of a compound comprising one or more LLT1 fragments.

Such LLT1 comprising compounds are defined hereafter. Advantageously, the presence of more than one LLT1 fragments in said compounds increases the potency of said compounds to modulate the activity of CD161 expressing cells.

In a particularly preferred embodiment of the above-defined method, CD161 expressing cells express the human CD161 receptor, in particular as represented by SEQ ID NO: 6.

The present invention also relates to a method as defined above, characterized in that the LLT1 expressing cells are NK cells, T cells, dendritic cells, macrophages, monocytes or B cells.

According to a preferred embodiment of the above-defined method the contacting of LLT1 expressing cells with CD161 or fragments thereof leads to the activation of said cells.

Under certain conditions the contacting of LLT1 expressing cells with CD161 or fragments thereof also leads to the inhibition of said cells.

According to another preferred embodiment of the above-defined method the LLT1 expressing cells are contacted with:
- a human CD161, in particular as represented by SEQ ID NO: 6, or
- a fragment of said human CD161 corresponding to the extracellular domains of CD161, in particular as represented by SEQ ID NO: 8.

According to another preferred embodiment of the above-defined method CD161, or fragments thereof, are presented at the surface of biological membranes, and in particular at the surface of cells, are used as soluble molecules, or are immobilized on a support.

In another preferred embodiment of the above-defined method the CD161 fragment is part of a compound comprising one or more CD161 fragments.

Advantageously, the presence of more than one CD161 fragments in said compound increases the potency of said compound to modulate the activity of LLT1 expressing cells.

In yet another preferred embodiment of the above-defined method, LLT1 expressing cells express the human LLT1 receptor, in particular as represented by SEQ ID NO: 2.

According to a particularly preferred embodiment of the above-defined method, LLT1 or fragments thereof, or CD161 or fragments thereof, may be modified by the insertion, the deletion or the substitution of at least one amino acid, provided that said modifications result in an improved CD161-LLT1 interaction, such as an enhanced binding capability or an improved stability. In particular, modified LLT1 or CD161 having an improved interaction stability at elevated temperatures, such as temperatures over 4°C, preferably over 20°C, and more preferably at approximately 37°C, are particularly preferred in the frame of the present invention.

The present invention also relates to a method for identifying, purifying, inactivating or destroying CD161 and/or LLT1 expressing cells, characterized in that said cells are contacted respectively with LLT1 or fragments thereof, and/or with CD161 or fragments thereof

Accordingly, for identifying CD161 and/or LLT1 expressing cells, respectively LLT1 or CD 161 will be advantageously linked to marker moieties such as fluorophores or haptens.

For purifying methods, LLT1 or fragments thereof, or CD161 or fragments thereof will be advantageously linked to a solid support or to marker moieties, so that the respective association of CD161 or LLT1 expressing cells leads to a complex which can be easily retrieved from a medium containing unpurified CD161 or LLT1 expressing cells.

For inactivating or destroying CD161 and/or LLT1 expressing cells, LLT1 or fragments thereof, or CD161 or fragments thereof will be advantageously linked to antibodies, or to toxic or cytolytic moieties. For destroying CD161 and/or LLT1 expressing cells, LLT1 or fragments thereof, or CD161 or fragments thereof will be advantageously linked to marker moieties such as fluorophores to visualize the cells and then destroy them by use of a laser.

The present invention also relates to a method for screening compounds liable to be used for the treatment of cancers, infectious diseases, such as viral, bacterial, or parasitic diseases, autoimmune diseases, inflammatory diseases, allergic reactions or organ and bone marrow transplantation rejection, characterized in that the compounds liable to be used for the treatment of the above mentioned pathologies are selected on their properties of inhibiting the binding between CD161 or fragments thereof, and LLT1 or fragments thereof.

Accordingly, a method for screening compounds with biological activity comprises providing cells expressing CD161 and/or LLT1 receptors at their cell surface, contacting the cells respectively with LLT1 or fragments thereof, and/or with CD161 or fragments thereof in the presence of the test compound, and determining whether the presence of the compound affects the binding of LLT1 or fragments thereof, and/or with CD161 or fragments thereof to CD161 and/or LLT1 expressing cells respectively. Preferably the cells expressing CD161 and/or LLT1 receptors in the method according to this aspect of the invention do not naturally express the receptors, and most preferably they are non-human cells. The cells are preferably stable transfectants, that is to say they contain nucleic acid material expressing CD161 and/or LLT1 stably integrated into their genome.

The present invention also relates to an isolated compound comprising at least two LLT1 molecules or fragments thereof.

The expression "isolated" relates to a compound comprising at least two LLT1 molecules or fragments thereof which is not part of a biological membrane. In particular, the isolated compound according to the invention is soluble.

In such a compound, the LLT1 molecules or fragments thereof are linked together, either directly or indirectly, via linker molecules, through covalent or non-covalent bonds.

According to a preferred embodiment of the isolated LLT1 comprising compound, said compound comprises LLT1 fragments as represented by SEQ ID NO: 4.

According to a another preferred embodiment of the isolated LLT1 comprising compound, the LLT1 fragments are linked to anchoring moieties, such as a γ-immunoglobulin Fc domain or biotin, and associated together via a linker molecule which binds to said anchoring molecules, such as protein A or an avidin protein.

Anchoring molecules may also be made of, or covered with a magnetic material or a biologically inert material, such as silicon, and be in the form of beads.

According to yet another preferred embodiment of the isolated LLT1 comprising compound, said compound also comprises:
- toxic or cytolytic moieties, such as toxins, radioactive molecules, recombinant active molecules or chemicals,
- marker moieties, such as fluorophores, magnetic beads, or haptens.

The present invention also relates to a pharmaceutical composition comprising as active substance, a compound liable to bind to CD161 or a compound liable to bind to LLT1, in association with a pharmaceutically acceptable carrier.

According to a preferred embodiment of the invention, the above-defined pharmaceutical composition comprises as active substance, a mammalian LLT1 or fragments thereof, as compounds liable to bind to CD161, or a mammalian CD161 or fragments thereof, as compounds liable to bind to LLT1, in association with a pharmaceutically acceptable carrier.

According to another preferred embodiment of the invention, the above-defined pharmaceutical composition comprises as active substance,
- a human LLT1, in particular as represented by SEQ ID NO: 2, or
- a fragment of said human LLT1 corresponding to the extracellular domain of LLT1, in particular as represented by SEQ ID NO: 4

According to another preferred embodiment of the invention, the above-defined pharmaceutical composition comprises as active substance an above-defined LLT1 comprising compound.

In another preferred embodiment, the present invention relates to an above-defined pharmaceutical composition comprising as active substance:
- a human CD161, in particular as represented by SEQ ID NO: 6, or
- a fragment of said human CD161 corresponding to the extracellular domain of CD161, in particular as represented by SEQ ID NO: 8.

In a particularly preferred embodiment, the present invention relates to an above-defined pharmaceutical composition, comprising as active substance an anti-CD161 or anti-LLT1 antibody, in particular a humanized anti-CD161 or anti-LLT1 antibody.

Such antibodies may be monoclonal or polyclonal antibodies, which can be easily obtained according to methods well-known to the man skilled in the art. In particular, monoclonal antibodies can be produced from hybridomas. Such hybridomas can be obtained by fusion of an antibody secreting cell, such as a B cell, with an immortalized cell, according to Kohler and Milstein. Nature. 256:495-497, 1975 or to Buttin et al. Curr. Top. Microbiol. Immun. 81:27-36,1978, for example.

As intended herein, the fragments of such antibodies, such as Fab, F(ab)'₂ or scFv fragments, are also considered as antibodies.

Humanized antibodies can be for example chimeric antibodies, in which, if appropriate, constant parts of animal antibodies, such as mouse or rabbit antibodies, are replaced by the corresponding parts of human antibodies, such as the Fc fragment for instance (Sharon et al, Nature. 309:364-367, 1984; Neuberger et al, Nature. 314:268-271, 1985). Alternatively, the complex determining region (CDR) of the animal antibodies can be grafted onto human antibodies, such as described for instance in US patent number 5,824,307, a process called "antibody reshaping" (Jones et al. Nature, 321:522-525, 1986). Another alternative technique is to produce human antibodies in mice using transgenic and transomic animals (Bruggemann et al, PNAS. 86:6709-6713, 1989; Richa et al, Science. 245:175-177, 1989; Capecchi, Trends in Genetics. 5:70-76, 1989).

The present invention also relates to a pharmaceutical composition comprising as active substance, single or multiple stranded nucleic acids encoding mammalian, in particular human, LLT1 or fragments thereof, or their complementary strands in the case of single stranded nucleic acids, in particular as represented respectively by SEQ ID NO: 1 and SEQ ID NO: 3, optionally comprised in a mammalian expression vector, in association with a pharmaceutically acceptable carrier.

The present invention also relates to a pharmaceutical composition comprising as active substance, single or multiple stranded nucleic acids encoding mammalian, in particular human, CD 161 or fragments thereof, or their complementary strands in the case of single stranded nucleic acids, in particular as represented respectively by SEQ ID NO: 5 and SEQ ID NO: 7, optionally comprised in a mammalian expression vector, in association with a pharmaceutically acceptable carrier.

Such nucleic acids or vectors are useful for various purposes, such as:
- the *in vivo, ex vivo or in vitro* production of the proteins they encode, *i.e.* LLT1 or fragments thereof and CD 161 or fragments thereof;
- the *in vivo, ex vivo* or *in vitro* production or delivery of antisens nucleic acids, which are liable to inhibit the expression of CD161 or LLT1;
- the *in vivo, ex vivo* or *in vitro* production or delivery of interference nucleic acids, such as siRNA, which are liable to inhibit the expression of CD161 or LLT1.
   The present invention also relates to the use of a compound liable to bind to CD161 or a compound liable to bind to LLT1, for the manufacture of a medicament intended for the prevention or the treatment of cancers, infectious diseases, such as viral, bacterial, or parasitic diseases, autoimmune diseases, inflammatory diseases, allergic reactions or organ and bone marrow transplantation rejection.
   In a preferred embodiment, the present invention relates to the above-defined use of a mammalian LLT1 or fragments thereof, as compounds liable to bind to CD161, or of a mammalian CD161 or fragments thereof, as compounds liable to bind to LLT1.
   In another preferred embodiment, the invention relates to the above-defined use, of
- a human LLT1, in particular as represented by SEQ ID NO: 2, or
- a fragment of said human LLT1 corresponding to the extracellular domain of LLT1, in particular as represented by SEQ ID NO: 4.

In a particularly preferred embodiment the present invention relates to the above-defined use, of a LLT1 comprising compound as defined above.

In another embodiment, the present invention relates to the above-defined use, of:
- a human CD161, in particular as represented by SEQ ID NO: 6, or
- a fragment of said human CD161 corresponding to the extracellular domain of CD161, in particular as represented by SEQ ID NO: 8.

In a preferred embodiment, the invention also relates to the above-defined use, of an anti-CD161 or anti-LLT1 antibody, in particular a humanized anti-CD161 or anti-LLT1 antibody.

The present invention also relates to the use of single or multiple stranded nucleic acids encoding mammalian, in particular human, LLT1 or fragments thereof, or their complementary strands in the case of single stranded nucleic acids, in particular as represented respectively by SEQ ID NO: 1 and SEQ ID NO: 3, optionally comprised in a mammalian expression vector, for the manufacture of a medicament intended for the prevention or the treatment of cancers, infectious diseases, such as viral, bacterial, or parasitic diseases, autoimmune diseases, inflammatory diseases, allergic reactions or organ and bone marrow transplantation rejection.

The present invention also relates to the use of single or multiple stranded nucleic acids encoding mammalian, in particular human, CD161 or fragments thereof, or their complementary strands in the case of single stranded nucleic acids, in particular as represented respectively by SEQ ID NO: 5 and SEQ ID NO: 7, optionally comprised in a mammalian expression vector, in association with a pharmaceutically acceptable carrier, for the manufacture of a medicament intended for the prevention or the treatment of cancers, infectious diseases, such as viral, bacterial, or parasitic diseases, autoimmune diseases, inflammatory diseases, allergic reactions or organ and bone marrow transplantation rejection.

### DESCRIPTION OF THE FIGURES

### Figure 1A and Figure 1B

### Production of chimeric LLT1-Fc fusion protein

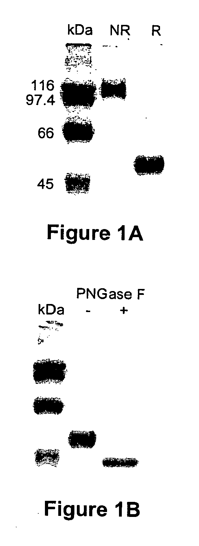

Figure 1A: Purified LLT1-Fc fusion protein was visualized on a 10% SDS-PAGE gel using reducing and nonreducing conditions yielding a ~55kDa and ~110 kDa protein respectively.

Figure 1B: Treatment of purified LLT1-Fc with peptide N-glycosidase F (PNGase F) yielded a ~48kDa protein.

### Figure 2

### LLT1 interacts with CD161 receptor

LLT1 multimer were generated using LLT1-Fc dimer conjugated to protein A-biotin and streptavidin-APC. Saturating amount of purified human IgG were added to block the remaining free protein A sites. A multimer control was generated by incubating ProteinA-biotin with saturing amount of purified human IgG.

LLT1 and Ctrl multimers were used to stain HEK293T cell untransfected or transfected with CD161-pIRES2-EGFP or LLT1-pIRES2-EGFP vectors.

LLT1 multimer bound to HEK293T cells transfected with CD161 but not to HEK293T expressing LLT1 or untransfected cells. Control multimer did not bind to any of these cells.

### Figure 3A and Figure 3B

### LLT1 multimer staining to CD161+ cells is inhibited by anti-CD161 antibodies

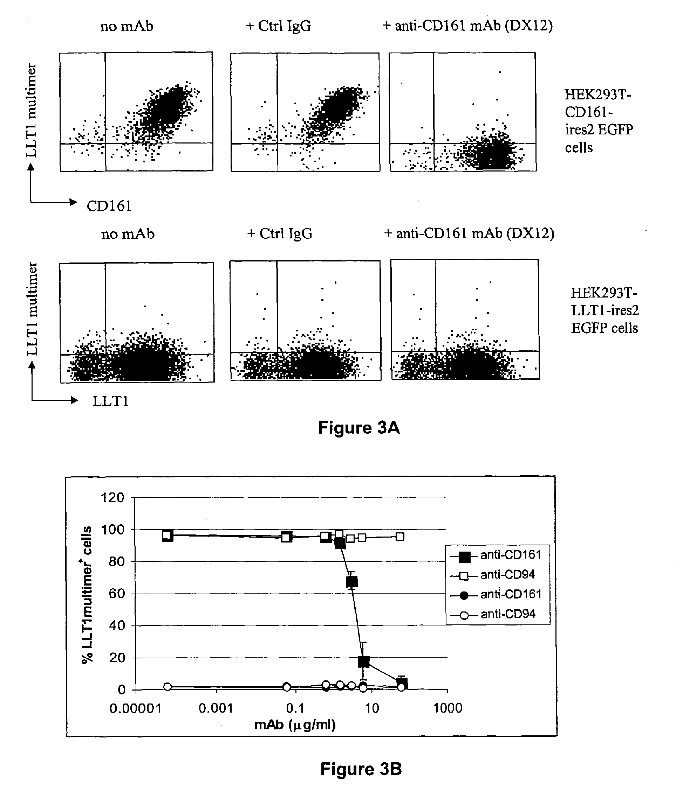

Figure 3A: LLT1 multimer was used to stain HEK293T cells transfected with CD161-pIRES2-EGFP or LLT1-pIRES2-EGFP vectors in the absence or presence of 3µg of irrelevant isotype IgG control (DX22) or anti-CD161 antibody (DX12). Similar blocking was seen using 191B8 anti-CD161 antibody.

Figure 3B: LLT1 multimer was used to stain HEK293T cells transfected with CD161-pIRES2-EGFP (squares) or LLT1-pIRES2-EGFP (circles) vectors in the presence of increasing concentration of irrelevant isotype IgG control (DX22) (open symbols) or anti-CD161 antibody (DX12) (closed symbols).

### Figure 4A and Figure 4B

### Interaction of polyclonal CD161⁺ NK and T cells with target cells expressing LLT1 results in a selective downregulation of CD161 on NK and T cells

HEK293T cells and Hela cells transfected or not with LLT1 were incubated with a polyclonal human NK and T cell population for 2 and 4 hours in complete medium at 37°C (ratio 1:1).

Cells were fixed and stained with anti-CD161 antibodies to monitor CD161 level of expression at the cell surface of NK and T cells.

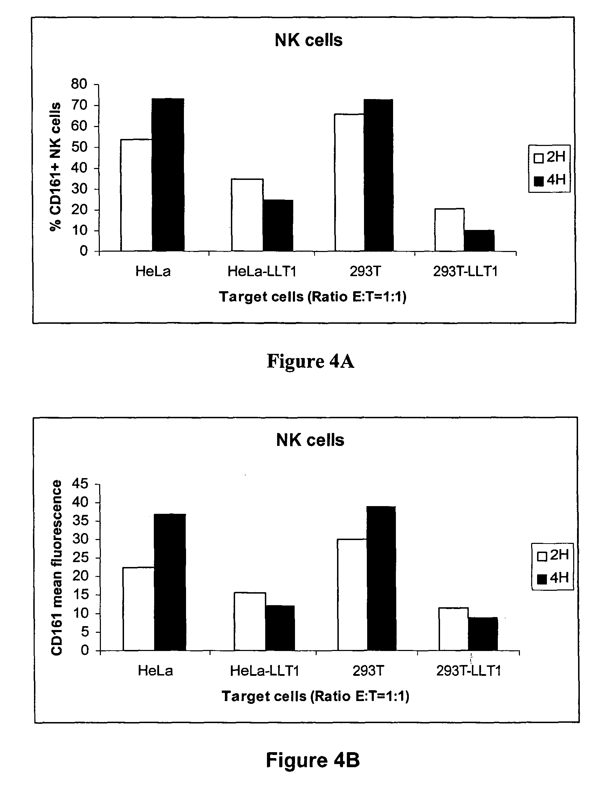

Figure 4A: % of CD161⁺ NK cells incubated with the indicated targets for 2 and 4 hours.

Figure 4B: level of CD161 expression (mean fluorescence) on NK cells incubated with the indicated targets for 2 and 4 hours.

Similar downregulation of CD161 on T cells is observed.

### Figure 5

### LLT1-induced downregulation of CD161 is blocked by anti-CD161 antibodies

Hela cells transfected or not with LLT1 were incubated with a polyclonal human NK and T cell population for 2 hours in complete medium at 37°C (ratio 1:1) in the presence of anti-CD161 (DX12) mAb or isotype IgG control (5µg/ml). Cells were fixed and stained with anti-CD161 antibody 191B8 (which recognizes a different epitope to DX12) to monitor CD161 level of expression at the cell surface of NK and T cells. % of CD161+ NK cells is shown and similar blocking is observed on T cells (data not shown). Identical blocking of LLT1-induced downregulation of CD161 is detected when anti-CD161 mAb 191B8 is used to block and anti-CD161 mAb DX12 is used to stain cells (data not shown).

### Figure 6

### LLT1-induced downregulation of CD 161 on NK and T cells needs cell-to-cell contacts

HEK293T cells transfected or not with LLT1 were incubated either in normal wells or transwells with a polyclonal human NK and T cell population for 4 hours in complete medium at 37°C (ratio 1:1). CD161 level of expression on NK and T cells was monitored using anti-CD 161 mAb.

### Figure 7

### Expression of LLT1 in HEK293T cells partially inhibits polyclonal IL-2 activated NK cell-mediated cytotoxicity.

293T cells transfected or not with LLT1 were labelled with ⁵¹chromium and incubated with polyclonal IL-2 activated NK cells for 18 hours at an E:T ratio of 20:1. Release of ⁵¹chromium was measured in the supernatant and % of specific lysis of the targets calculated.

### EXAMPLES

### Example 1

### Construction of LLT1 multimeric complexes

Extracellular domains (residues 60-191) (SEQ ID NO: 4) of LLT1 (AF133299, Q9UHP7) (SEQ ID NO: 2) were fused to the human IgG1 FC portion and the chimeric protein produced in transfected eukaryotic cells. The soluble fusion LLT1-Fc protein secreted in the media by the transfected cells was purified on protein-A-Sepharose column and conjugated to proteinA-biotin to generate a multimer. This multimer was labelled with a fluorescent marker using Streptavidin-APC.

This multimer can then be used to visualise interactions between LLT1 and cell surface receptors.

### Method

LLT1 AA sequence (191 amino acid residues) (AF133299, Q9UHP7):

MHDSNNVEKDITPSELPANPGCLHSKEHSIKATLIWRLFFLIM
FLTIIVCGMVAALSAIRANCHQEPSVCLQAACPESWIGFQRK
CFYFSDDTKNWTSSQRFCDSQDADLAQVESFQELNFLLRYK
GPSDHWIGLSREQGQPWKWINGTEWTRQFPILGAGECAYLN
DKGASSARHYTERKWICSKSDIHV (SEQ ID NO: 2)

Amino acid residues underlined were removed in the LLT1-Fc chimeric molecule.

Full length LLT1 cDNA was amplified by reverse transcription (RT)-PCR from polyclonal NK total RNA extracted with TRIzol Reagent (15596-26, Invitrogen) using an oligo(dT) primer for the RT and the primers LLT1-F (5'-ATGCATGACAGTAACAATGTGG-3') (SEQ ID NO: 9) and LLT1-R (5'-TAGTTGGGGCTTTGCTGTAA-3') (SEQ ID NO: 10) for the PCR (95°C for 2min, 35 cycles with 95°C for 45 sec, 60°C for 45 sec, 72°C for 1 min and a final elongation at 72°C for 10 min). The PCR product was cloned into pGEMT-easy vector (A1360, Promega).

Codons corresponding to the extracellular domains (60-191) of LLT1 were amplified by PCR using full length LLT1 cDNA as a template and the following primers:

FLLT1BamHI-    5'-AAGGGGATCCGAGAGCTAACTGCCATCAAGAGCC-3'  (SEQ  ID
NO: 11)

RLLT1NotI-     5'-CTCGAGCGGCCGCCTAGACATGTATATCTGATTTGG-3'  (SEQ  ID
NO: 12)

The PCR product was subcloned into pcDNA3 vector in frame with the signal sequence of human CD5 and the Fc portion of human IgGl. The final construct was sequenced.

LLT1-Fc dimers were generated by transiently transfecting eukaryotic HEK293T cells. The chimeric protein was released in the media and purified onto a ProteinA-Sepharose column. Purity was assessed by SDS-PAGE electrophoresis (**Figure 1A**). Peptide N-glycosidase F (PNGase **F**) treatment was performed according to the manufactor instruction (New England Biolabs) (**Figure 1B**). Briefly, 5 µg of purified LLT1-Fc was denatured in 0.5 % SDS, 1% β-mercaptoethanol denaturing buffer at 100°C for 10 minutes, and digested with 2000 Unit of PNGase F in 1 % NP40, 50 mM sodium phosphate pH 7.5 for 1 hour at 37°C.

A multimeric complex was generated by incubating the dimers with ProteinA-biotin (Pierce) at a ratio of 10:1 for 30 minutes at room temperature. Saturating amount of purified human IgG were added to block the remaining free protein A sites. These complexes were conjugated with Streptavidin-APC to generate a fluorescent reagent that can be used in flow cytometry analysis.

A multimer control was generated by incubating ProteinA-biotin with saturing amount of purified human IgG.

### Example 2

### Binding of LLT1 multimer

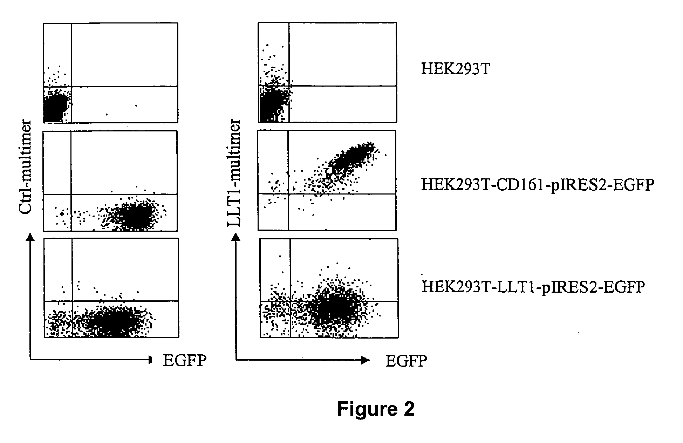

Flow cytometry using the LLT1-Fc-ProteinA-biotin-Streptavidin-APC multimer (LLT1-multimer) was performed to identify the receptor of LLT1. LLT1-multimer stained HEK293T cells stably transfected with human CD161 (U11276, Q12918) cloned into pIRES2-EGFP (clontech) while it did not stain untransfected HEK293T or HEK293T stably transfected with LLT1-pIRES2-EGFP **(Figure 2**). A control multimer did not stain any of the cells. The specificity of the binding of LLT1-multimer to CD161 was further confirmed by addition of anti-CD161 antibodies which abolished the staining while irrelevant isotype control antibodies did not (**Figures 3A and 3B**).

### Example 3

### Interaction of LLT1 with CD161 induces a downregulation of CD161

Human polyclonal NK cells were isolated from peripheral mononuclear cells of healthy donors by positive selection using CD56 MicroBeads (Miltenyi Biotec). NK cells were then stimulated with PHA-P (1 ug/ml, SIGMA), irradiated PBMC and B-EBV feeder cells in Iscove's Modification-ATL medium (biowest) supplemented with L-glutamine (2mM, Gibco), Penicillin-Streptomycin (Gibco) and rIL-2 (500 U/ml).

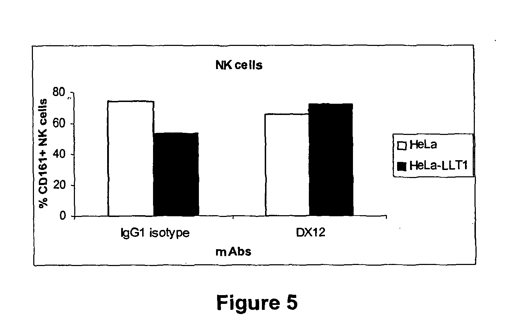

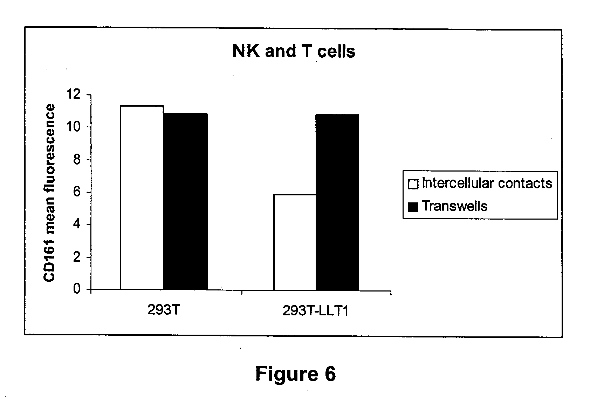

HEK293T cells and Hela cells transfected or not with LLT1 were incubated with a polyclonal human NK and T cell population for 10 minutes up to 4 hours in complete medium at 37°C. Cells were fixed and stained with anti-CD161 antibodies to monitor CD161 level of expression at the cell surface of NK and T cells. A specific and significant loss of CD161⁺ cells is observed when NK and T cells are incubated with cells expressing LLT1 and not with untransfected cells (**Figures 4A and 4B**). This loss is observed as early as 10 minutes and is abolished by addition of anti-CD161 mAbs (**Figure 5**). This loss of CD161⁺ NK and T cells is not due to release of soluble form of LLT1 blocking binding of anti-CD161 antibodies as no effect on CD161 expression was observed when the incubation was performed using transwells (**Figure 6**). This later result therefore demonstrates that LLT1 induces a downregulation of the CD161 receptor.

### Exemple 4

### Expression of LLT1 in 293T cells partially inhibits polyclonal IL-2 activated NK cell-mediated cytotoxicity

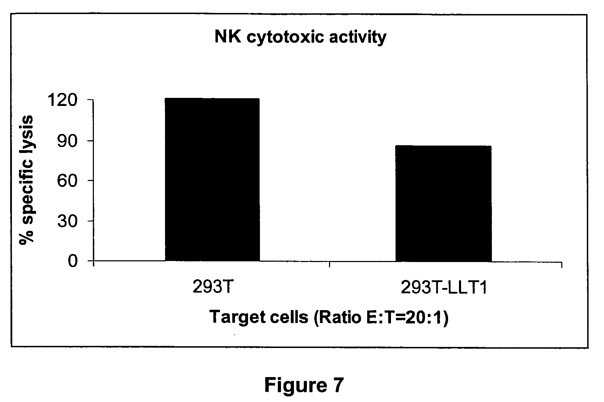

293T cells transfected or not with LLT1 were labelled with ⁵¹chromium and incubated with polyclonal IL-2 activated NK cells for 18 hours at an E:T ratio of 20:1. Release of ⁵¹chromium was measured in the supernatant and specific lysis of the targets calculated (**Figure 7**). A 28.3 % lysis decrease was observed for LLT1 expressing cells with respect to control cells.

## Claims

1. A method for modulating *in vivo* or *in vitro* the activity of cells expressing CD161 and/or LLT1, **characterized in that** said cells are contacted respectively with LLT1 or fragments thereof, and/or with CD 161 or fragments thereof.

2. A method according to claim 1, **characterized in that** the CD161 expressing cells are NK cells, CD1d restricted Vα24⁺ NKT cells, or T cells, such as α*β* T cells or γ*δ* T cells.

3. A method according to claim 1 or 2, **characterized in that** when the CD161 expressing cells are NK cells, the contacting of said cells with LLT1 or fragments thereof leads to the inhibition of said cells.

4. A method according to claim 1 or 2, **characterized in that** when the CD161 expressing cells are T cells or CD1d restricted Vα24⁺ NKT cells, the contacting of said cells with LLT1 or fragments thereof leads to the activation of said cells.

5. A method according to any of claims 1 to 4, **characterized in that** the CD161 expressing cells are contacted with:
- a human LLT1, in particular as represented by SEQ ID NO: 2, or
- a fragment of said human LLT1 corresponding to the extracellular domains of LLT1, in particular as represented by SEQ ID NO: 4.

6. A method according to any of claims 1 to 5, **characterized in that** LLT1, or fragments thereof, are presented at the surface of biological membranes, and in particular at the surface of cells, are used as soluble molecules, or are immobilized on a support.

7. A method according to any of claims 1 to 5, **characterized in that** the LLT1 fragment is part of a compound comprising one or more LLT1 fragments.

8. A method according to any of claims 1 to 7, **characterized in that** CD161 expressing cells express the human CD 161 receptor, in particular as represented by SEQ ID NO: 6.

9. A method according to claim 1, **characterized in that** the LLT1 expressing cells are NK cells, T cells, dendritic cells, macrophages, monocytes or B cells.

10. A method according to claim 9, **characterized in that** the contacting of LLT1 expressing cells with CD 161 or fragments thereof leads to the activation of said cells.

11. A method according to any of claims 9 to 10, **characterized in that** the LLT1 expressing cells are contacted with:
- a human CD 161, in particular as represented by SEQ ID NO: 6, or
- a fragment of said human CD161 corresponding to the extracellular domains of CD161, in particular as represented by SEQ ID NO: 8.

12. A method according to any of claims 9 to 11, **characterized in that** CD161, or fragments thereof, are presented at the surface of biological membranes, and in particular at the surface of cells, are used as soluble molecules, or are immobilized on a support.

13. A method according to any of claims 9 to 12, **characterized in that** the CD161 fragment is part of a compound comprising one or more CD161 fragments.

14. A method according to any of claims 9 to 13, **characterized in that** LLT1 expressing cells express the human LLT1 receptor, in particular as represented by SEQ ID NO: 2.

15. A method for identifying, purifying, inactivating or destroying CD161 and/or LLT1 expressing cells, **characterized in that** said cells are contacted respectively with LLT1 or fragments thereof, and/or with CD161 or fragments thereof.

16. A method for screening compounds liable to be used for the treatment of cancers, infectious diseases, such as viral, bacterial, or parasitic diseases, autoimmune diseases, inflammatory diseases, allergic reactions or organ and bone marrow transplantation rejection, **characterized in that** the compounds liable to be used for the treatment of the above mentioned pathologies are selected on their properties of inhibiting the binding between CD161 or fragments thereof, and LLT1 or fragments thereof.

17. An isolated compound comprising at least two LLT1 molecules or fragments thereof.

18. An isolated compound according to claim 17, comprising LLT1 fragments as represented by SEQ ID NO: 4.

19. An isolated compound according to claim 17 or 18, wherein the LLT1 fragments are linked to anchoring moieties, such as a γ-immunoglobulin Fc domain or biotin, and associated together via a linker molecule which binds to said anchoring molecules, such as protein A or an avidin protein.

20. An isolated compound according to any of claims 17 to 19, wherein said compound also comprises:
- toxic or cytolytic moieties, such as toxins, radioactive molecules, recombinant active molecules or chemicals,
- marker moieties, such as fluorophores, magnetic beads, or haptens.

21. A pharmaceutical composition comprising as active substance, a compound liable to bind to CD161 or a compound liable to bind to LLT1, in association with a pharmaceutically acceptable carrier.

22. A pharmaceutical composition according to claim 21 comprising as active substance, a mammalian LLT1 or fragments thereof, as compounds liable to bind to CD161, or a mammalian CD161 or fragments thereof, as compounds liable to bind to LLT1, in association with a pharmaceutically acceptable carrier.

23. A pharmaceutical composition according to claim 21 or 22, comprising as active substance,
- a human LLT1, in particular as represented by SEQ ID NO: 2, or
- a fragment of said human LLT1 corresponding to the extracellular domain of LLT1, in particular as represented by SEQ ID NO: 4

24. A pharmaceutical composition according to any of claims 21 to 23, comprising as active substance a compound according to any of claims 17 to 20.

25. A pharmaceutical composition according to claim 21 or 22, comprising as active substance:
- a human CD161, in particular as represented by SEQ ID NO: 6, or
- a fragment of said human CD161 corresponding to the extracellular domain of CD161, in particular as represented by SEQ ID NO: 8.

26. A pharmaceutical composition according to claim 21, comprising as active substance an anti-CD161 or anti-LLT1 antibody, in particular a humanized anti-CD161 or anti-LLT1 antibody.

27. A pharmaceutical composition comprising as active substance, single or multiple stranded nucleic acids encoding mammalian, in particular human, LLT1 or fragments thereof, or their complementary strands in the case of single stranded nucleic acids, in particular as represented respectively by SEQ ID NO: 1 and SEQ ID NO: 3, optionally comprised in a mammalian expression vector, in association with a pharmaceutically acceptable carrier.

28. A pharmaceutical composition comprising as active substance, single or multiple stranded nucleic acids encoding mammalian, in particular human, CD161 or fragments thereof, or their complementary strands in the case of single stranded nucleic acids, in particular as represented respectively by SEQ ID NO: 5 and SEQ ID NO: 7, optionally comprised in a mammalian expression vector, in association with a pharmaceutically acceptable carrier.

29. The use of a compound liable to bind to CD161 or a compound liable to bind to LLT1, for the manufacture of a medicament intended for the prevention or the treatment of cancers, infectious diseases, such as viral, bacterial, or parasitic diseases, autoimmune diseases, inflammatory diseases, allergic reactions or organ and bone marrow transplantation rejection.

30. The use according to claim 29, of a mammalian LLT1 or fragments thereof, as compounds liable to bind to CD161, or of a mammalian CD161 or fragments thereof, as compounds liable to bind to LLT 1.

31. The use according to claim 29 or 30, of
- a human LLT1, in particular as represented by SEQ ID NO: 2, or
- a fragment of said human LLT1 corresponding to the extracellular domain of LLT1, in particular as represented by SEQ ID NO: 4.

32. The use according to any of claims 29 to 31, of a compound according to any of claims 17 to 20.

33. The use according to claim 29 or 30, of:
- a human CD161, in particular as represented by SEQ ID NO: 6, or
- a fragment of said human CD161 corresponding to the extracellular domain of CD161, in particular as represented by SEQ ID NO: 8.

34. The use according to claim 29, of an anti-CD161 or anti-LLT1 antibody, in particular a humanized anti-CD161 or anti-LLT1 antibody.

35. The use of single or multiple stranded nucleic acids encoding mammalian, in particular human, LLT1 or fragments thereof, or their complementary strands in the case of single stranded nucleic acids, in particular as represented respectively by SEQ ID NO: 1 and SEQ ID NO: 3, optionally comprised in a mammalian expression vector, for the manufacture of a medicament intended for the prevention or the treatment of cancers, infectious diseases, such as viral, bacterial, or parasitic diseases, autoimmune diseases, inflammatory diseases, allergic reactions or organ and bone marrow transplantation rejection.

36. The use of single or multiple stranded nucleic acids encoding mammalian, in particular human, CD 161 or fragments thereof, or their complementary strands in the case of single stranded nucleic acids, in particular as represented respectively by SEQ ID NO: 5 and SEQ ID NO: 7, optionally comprised in a mammalian expression vector, in association with a pharmaceutically acceptable carrier, for the manufacture of a medicament intended for the prevention or the treatment of cancers, infectious diseases, such as viral, bacterial, or parasitic diseases, autoimmune diseases, inflammatory diseases, allergic reactions or organ and bone marrow transplantation rejection.


SEQUENCE LISTING

<110> CENTRE NATIONAL DE LA RECHERCHE SCIENTIFIQUE (CNRS)

<120> METHOD FOR MODULATING THE ACTIVITY OF CELLS OF THE IMMUNE SYSTEM

<130> IOB 04 CK CNR LLT1

<160> 12

<170> PatentIn version 3.1

<210> 1
<211> 576
<212> DNA
<213> Homo sapiens

<400> 1
atgcatgaca gtaacaatgt ggagaaagac attacaccat ctgaattgcc tgcaaaccca 60

ggttgtctgc attcaaaaga gcattctatt aaagctacct taatttggcg cttatttttc 120

ttaatcatgt ttctgacaat catagtgtgt ggaatggttg ctgctttaag cgcaataaga 180

gctaactgcc atcaagagcc atcagtatgt cttcaagctg catgcccaga aagctggatt 240

ggttttcaaa gaaagtgttt ctatttttct gatgacacca agaactggac atcaagtcag 300

aggttttgtg actcacaaga tgctgatctt gctcaggttg aaagcttcca ggaactgaat 360

ttcctgttga gatataaagg cccatctgat cactggattg ggctgagcag agaacaaggc 420

caaccatgga aatggataaa tggtactgaa tggacaagac agtttcctat cctgggagca 480

ggagagtgtg cctatttgaa tgacaaaggt gccagtagtg ccaggcacta cacagagagg 540

aagtggattt gttccaaatc agatatacat gtctag 576

<210> 2
<211> 191
<212> PRT
<213> Homo sapiens

<400> 2

Met His Asp Ser Asn Asn Val Glu Lys Asp Ile Thr Pro Ser Glu Leu
1               5                   10                  15

Pro Ala Asn Pro Gly Cys Leu His Ser Lys Glu His Ser Ile Lys Ala
            20                  25                  30

Thr Leu Ile Trp Arg Leu Phe Phe Leu Ile Met Phe Leu Thr Ile Ile
        35                  40                  45

Val Cys Gly Met Val Ala Ala Leu Ser Ala Ile Arg Ala Asn Cys His
    50                  55                  60

Gln Glu Pro Ser Val Cys Leu Gln Ala Ala Cys Pro Glu Ser Trp Ile
65                  70                  75                  80

Gly Phe Gln Arg Lys Cys Phe Tyr Phe Ser Asp Asp Thr Lys Asn Trp
                85                  90                  95

Thr Ser Ser Gln Arg Phe Cys Asp Ser Gln Asp Ala Asp Leu Ala Gln
            100                 105                 110

Val Glu Ser Phe Gln Glu Leu Asn Phe Leu Leu Arg Tyr Lys Gly Pro
        115                 120                 125

Ser Asp His Trp Ile Gly Leu Ser Arg Glu Gln Gly Gln Pro Trp Lys
    130                 135                 140

Trp Ile Asn Gly Thr Glu Trp Thr Arg Gln Phe Pro Ile Leu Gly Ala
145                 150                 155                 160

Gly Glu Cys Ala Tyr Leu Asn Asp Lys Gly Ala Ser Ser Ala Arg His
                165                 170                 175

Tyr Thr Glu Arg Lys Trp Ile Cys Ser Lys Ser Asp Ile His Val
            180                 185                 190

<210>  3
<211>  399
<212>  DNA
<213>  Artificial sequence

<220>
<223>  LLT1 fragment

<400>  3
agagctaact gccatcaaga gccatcagta tgtcttcaag ctgcatgccc agaaagctgg      60

attggttttc aaagaaagtg tttctatttt tctgatgaca ccaagaactg gacatcaagt     120

cagaggtttt gtgactcaca agatgctgat cttgctcagg ttgaaagctt ccaggaactg     180

aatttcctgt tgagatataa aggcccatct gatcactgga ttgggctgag cagagaacaa     240

ggccaaccat ggaaatggat aaatggtact gaatggacaa gacagtttcc tatcctggga     300

gcaggagagt gtgcctattt gaatgacaaa ggtgccagta gtgccaggca ctacacagag     360

aggaagtgga tttgttccaa atcagatata catgtctag                            399

<210>  4
<211>  132
<212>  PRT
<213>  Artificial sequence

<220>
<223>  LLT1 fragment

<400>  4

Arg Ala Asn Cys His Gln Glu Pro Ser Val Cys Leu Gln Ala Ala Cys
1               5                   10                  15

Pro Glu Ser Trp Ile Gly Phe Gln Arg Lys Cys Phe Tyr Phe Ser Asp
            20                  25                  30

Asp Thr Lys Asn Trp Thr Ser Ser Gln Arg Phe Cys Asp Ser Gln Asp
        35                  40                  45

Ala Asp Leu Ala Gln Val Glu Ser Phe Gln Glu Leu Asn Phe Leu Leu
    50                  55                  60

Arg Tyr Lys Gly Pro Ser Asp His Trp Ile Gly Leu Ser Arg Glu Gln
65                  70                  75                  80

Gly Gln Pro Trp Lys Trp Ile Asn Gly Thr Glu Trp Thr Arg Gln Phe
                85                  90                  95

Pro Ile Leu Gly Ala Gly Glu Cys Ala Tyr Leu Asn Asp Lys Gly Ala
            100                 105                 110

Ser Ser Ala Arg His Tyr Thr Glu Arg Lys Trp Ile Cys Ser Lys Ser
        115                 120                 125

Asp Ile His Val
    130

<210>  5
<211>  678
<212>  DNA
<213>  Homo sapiens

<400>  5
atggaccaac aagcaatata tgctgagtta aacttaccca cagactcagg cccagaaagt      60

tcttcacctt catctcttcc tcgggatgtc tgtcagggtt caccttggca tcaatttgcc     120

ctgaaactta gctgtgctgg gattattctc cttgtcttgg ttgttactgg gttgagtgtt     180

tcagtgacat ccttaataca gaaatcatca atagaaaaat gcagtgtgga cattcaacag     240

agcaggaata aaacaacaga gagaccgggt ctcttaaact gcccaatata ttggcagcaa     300

ctccgagaga aatgcttgtt attttctcac actgtcaacc cttggaataa cagtctagct     360

gattgttcca ccaaagaatc cagcctgctg cttattcgag ataaggatga attgatacac     420

acacagaacc tgatacgtga caaagcaatt ctgttttgga ttggattaaa tttttcatta     480

tcagaaaaga actggaagtg gataaacggc tcttttttaa attctaatga cttagaaatt     540

agaggtgatg ctaaagaaaa cagctgtatt tccatctcac agacatctgt gtattctgag     600

tactgtagta cagaaatcag atggatctgc caaaaagaac taacacctgt gagaaataaa     660

gtgtatcctg actcttga                                                   678

<210>  6
<211>  225
<212>  PRT
<213>  Homo sapiens

<400>  6

Met Asp Gln Gln Ala Ile Tyr Ala Glu Leu Asn Leu Pro Thr Asp Ser
1               5                   10                  15

Gly Pro Glu Ser Ser Ser Pro Ser Ser Leu Pro Arg Asp Val Cys Gln
            20                  25                  30

Gly Ser Pro Trp His Gln Phe Ala Leu Lys Leu Ser Cys Ala Gly Ile
        35                  40                  45

Ile Leu Leu Val Leu Val Val Thr Gly Leu Ser Val Ser Val Thr Ser
    50                  55                  60

Leu Ile Gln Lys Ser Ser Ile Glu Lys Cys Ser Val Asp Ile Gln Gln
65                  70                  75                  80

Ser Arg Asn Lys Thr Thr Glu Arg Pro Gly Leu Leu Asn Cys Pro Ile
                85                  90                  95

Tyr Trp Gln Gln Leu Arg Glu Lys Cys Leu Leu Phe Ser His Thr Val
            100                 105                 110

Asn Pro Trp Asn Asn Ser Leu Ala Asp Cys Ser Thr Lys Glu Ser Ser
        115                 120                 125

Leu Leu Leu Ile Arg Asp Lys Asp Glu Leu Ile His Thr Gln Asn Leu
    130                 135                 140

Ile Arg Asp Lys Ala Ile Leu Phe Trp Ile Gly Leu Asn Phe Ser Leu
145                 150                 155                 160

Ser Glu Lys Asn Trp Lys Trp Ile Asn Gly Ser Phe Leu Asn Ser Asn
                165                 170                 175

Asp Leu Glu Ile Arg Gly Asp Ala Lys Glu Asn Ser Cys Ile Ser Ile
            180                 185                 190

Ser Gln Thr Ser Val Tyr Ser Glu Tyr Cys Ser Thr Glu Ile Arg Trp
        195                 200                 205

Ile Cys Gln Lys Glu Leu Thr Pro Val Arg Asn Lys Val Tyr Pro Asp
    210                 215                 220

Ser
225

<210> 7
<211> 480
<212> DNA
<213> Artificial sequence

<220>
<223> CD161 fragment

<400> 7
cagaaatcat caatagaaaa atgcagtgtg gacattcaac agagcaggaa taaaacaaca 60

gagagaccgg gtctcttaaa ctgcccaata tattggcagc aactccgaga gaaatgcttg 120

ttattttctc acactgtcaa cccttggaat aacagtctag ctgattgttc caccaaagaa 180

tccagcctgc tgcttattcg agataaggat gaattgatac acacacagaa cctgatacgt 240

gacaaagcaa ttctgttttg gattggatta aatttttcat tatcagaaaa gaactggaag 300

tggataaacg gctctttttt aaattctaat gacttagaaa ttagaggtga tgctaaagaa 360

aacagctgta tttccatctc acagacatct gtgtattctg agtactgtag tacagaaatc 420

agatggatct gccaaaaaga actaacacct gtgagaaata aagtgtatcc tgactcttga 480

<210> 8
<211> 159
<212> PRT
<213> Artificial sequence

<220>
<223> CD161 fragment

<400> 8

Gln Lys Ser Ser Ile Glu Lys Cys Ser Val Asp Ile Gln Gln Ser Arg
1               5                   10                  15

Asn Lys Thr Thr Glu Arg Pro Gly Leu Leu Asn Cys Pro Ile Tyr Trp
            20                  25                  30

Gln Gln Leu Arg Glu Lys Cys Leu Leu Phe Ser His Thr Val Asn Pro
        35                  40                  45

Trp Asn Asn Ser Leu Ala Asp Cys Ser Thr Lys Glu Ser Ser Leu Leu
    50                  55                  60

Leu Ile Arg Asp Lys Asp Glu Leu Ile His Thr Gln Asn Leu Ile Arg
65                  70                  75                  80

Asp Lys Ala Ile Leu Phe Trp Ile Gly Leu Asn Phe Ser Leu Ser Glu
                85                  90                  95

Lys Asn Trp Lys Trp Ile Asn Gly Ser Phe Leu Asn Ser Asn Asp Leu
            100                 105                 110

Glu Ile Arg Gly Asp Ala Lys Glu Asn Ser Cys Ile Ser Ile Ser Gln
      115                 120                 125

Thr Ser Val Tyr Ser Glu Tyr Cys Ser Thr Glu Ile Arg Trp Ile Cys
   130                 135                 140

Gln Lys Glu Leu Thr Pro Val Arg Asn Lys Val Tyr Pro Asp Ser
145                 150                 155

<210> 9
<211> 22
<212> DNA
<213> Artificial sequence

<220>
<223> PCR primer

<400> 9
atgcatgaca gtaacaatgt gg                                              22

<210> 10
<211> 20
<212> DNA
<213> Artificial sequence

<220>
<223> PCR primer

<400> 10
tagttggggc tttgctgtaa                                                 20

<210> 11
<211> 34
<212> DNA
<213> Artificial sequence

<220>
<223> PCR primer

<400> 11
aaggggatcc gagagctaac tgccatcaag agcc                                 34

<210> 12
<211> 36
<212> DNA
<213> Artificial sequence

<220>
<223> PCR primer

<400> 12
ctcgagcggc cgcctagaca tgtatatctg atttgg                               36